(19) Europäisches Patentamt
European Patent Office
Office européen des brevets

(11) EP 1 275 382 A2

(12) # EUROPÄISCHE PATENTANMELDUNG

(43) Veröffentlichungstag:
**15.01.2003 Patentblatt 2003/03**

(51) Int Cl.[7]: **A61K 31/122**, A61K 35/78, A61P 35/00

(21) Anmeldenummer: **02022953.0**

(22) Anmeldetag: **24.11.1999**

(84) Benannte Vertragsstaaten:
**AT BE CH CY DE DK ES FI FR GB GR IE IT LI LU MC NL PT SE**

(30) Priorität: **25.11.1998 DE 19854446**
**24.03.1999 DE 19913333**

(62) Dokumentnummer(n) der früheren Anmeldung(en) nach Art. 76 EPÜ:
**99958118.4 / 1 131 063**

(71) Anmelder: **Universitätsklinikum Freiburg**
**79106 Freiburg (DE)**

(72) Erfinder:
- **Schempp, Christoph, Dr.**
  **79102 Freiburg (DE)**
- **Simon, Jan C., Prof. Dr.**
  **79249 Merzhausen (DE)**
- **Simon-Haarhaus, Birgit**
  **79194 Gundelfingen (DE)**
- **Schöpf, Erwin, Prof. Dr.**
  **79104 Freiburg (DE)**

(74) Vertreter: **Keller, Günter, Dr. et al**
**Lederer & Keller**
**Patentanwälte**
**Prinzregentenstrasse 16**
**80538 München (DE)**

Bemerkungen:
Diese Anmeldung ist am 11 - 10 - 2002 als Teilanmeldung zu der unter INID-Kode 62 erwähnten Anmeldung eingereicht worden.

(54) **Hyperforin als Zytostatikum sowie Hyperforin-Salbe oder -Creme als Applikationsform**

(57) Die Erfindung betrifft die Verwendung von Hyperforin zur Behandlung von Krebserkrankungen und/ oder Präkanzerosen. Außerdem betrifft die Erfindung eine Hyperforin-haltige Salbe oder Creme, deren Herstellung und Anwendung. Die Hyperforin-haltige Salbe oder Creme eignet sich auch zur Behandlung von entzündlichen Hauterkrankungen, Altershaut und bakteriellen Hauterkrankungen sowie Hauterkrankungen im Bereich der Veterinärmedizin.

EP 1 275 382 A2

**Beschreibung**

Die vorliegende Erfindung betrifft die Verwendung von Hyperforin als Zytostatikum sowie eine Hyperforin-Salbe oder -Creme, die sich als Applikationsform für das Hyperforin eignet.

Hyperforin zählt neben den Hyperizinen zu den charakteristischen Inhaltsstoffen des Johanniskrauts (Hypericum perforatum L.), das darüber hinaus allgemein im Pflanzenreich vorkommende Inhaltsstoffe wie Flavon- und Flavonolderivate, Rutin, Hyperosid, Xanthonderivate, Amentoflavon, Biapigenin und ätherische Öle enthält.

Johanniskraut und Johanniskrautauszüge werden schon seit geraumer Zeit in der Medizin und der Volksmedizin als Arzneimittel für verschiedenste Indikationen eingesetzt. Neuerdings wird der Bestandteil Hypericin als Wirkstoff auch isoliert in Arzneimitteln verwendet (L. Roth, Hypericum, Hypericin, Ecomed Arzneipflanzen-Monographie, Ecomed, Landsberg/Lech, 1990).

Die Monographie "Hyperici herba (Johanniskraut)", die von der Kommission E des früheren Gesundheitsamtes am 5.12.1984 publiziert wurde, nennt als Anwendungsgebiet für Hypericum-Präparate (intern als Tropfen oder Tabletten): "Psychovegetative Störungen, depressive Verstimmungszustände, Angst und/oder nervöse Unruhe". In zahlreichen plazebokontrollierten Studien wurde die den trizyklischen Antidepressiva vergleichbare antidepressive Wirksamkeit des Johanniskrauts belegt.

Als Hausmittel wird insbesondere das Johanniskrautöl zur Wundund Schmerzbehandlung und bei Verbrennungen eingesetzt (L. Roth a.a.O.).

Als Wirkstoff des Johanniskrautöls wurde zunächst aufgrund der charakteristischen roten Farbe und Fluoreszenz des Öls Hypericin angenommen. Die Formel von Hypericin ist im folgenden wiedergegeben:

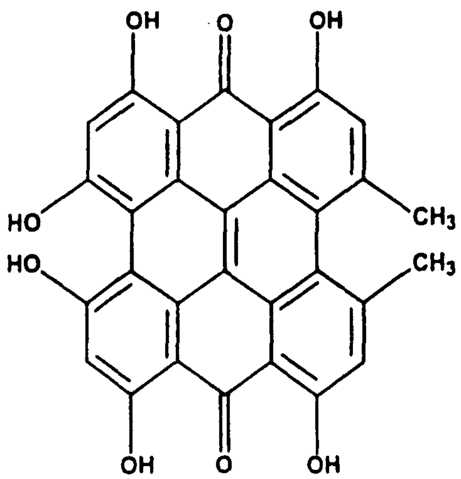

Neuere Untersuchungen der Zusammensetzung von Johanniskrautöl haben jedoch ergeben, daß es tatsächlich kein Hypericin enthält, sondern sog. Ölhypericine, welche lipophile Lyseprodukte des Hypericins darstellen. Außerdem enthält Johanniskrautöl Hyperforin (P. Maisenbacher et al., Planta Med. 58:351-354 (1992) und B. Hellwig, DAZ 137, 29, Seiten 35-36), dessen Formel im folgenden wiedergegeben ist:

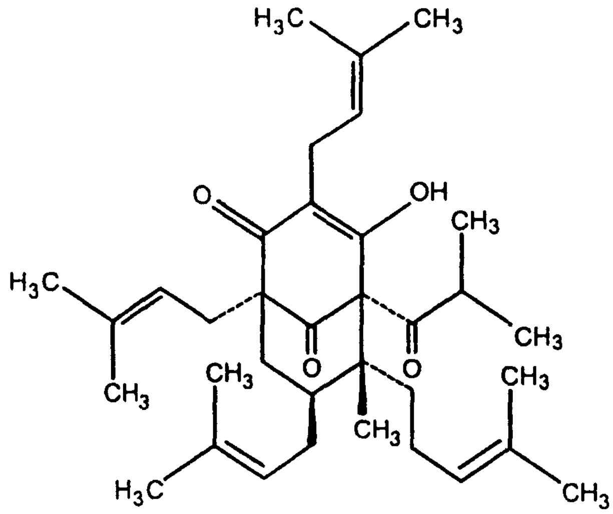

Hyperforin hat als Wirkstoff Interesse als antidepressive Substanz erweckt (Pharmacopsychiatry 1998, Vol. 31, Supplement 1, Seiten 1-60). Darüberhinaus ist Hyperforin antibakteriell wirksam (A. I. Gurevich et al., L. Antibiotiki, 16: 510-513 (1971)).

Stabile Extrakte aus Hypericum perforatum L. sind aus der DE 197 14 450 und der DE 196 46 977 bekannt.

Die DE 195 47 317 offenbart ein antivirales Medikament auf der Basis von Wirkstoffen des Johanniskrauts, das 1-50% Hypericin oder Pseudohypericin enthält.

Die EP-A-0 599 307 offenbart einen Trockenextrakt aus Johanniskraut mit erhöhtem Hyperforin-Gehalt sowie dessen Verwendung zur Herstellung psychovegetativ und antidepressiv wirksamer Arzneimittel.

Neben Johanniskrautöl sind Salben bekannt, die neben verschiedenen anderen Heilkräutern Hypericum in geringer Konzentration enthalten. Hierzu gehören beispielsweise "Unguentum Truw" zur Wundbehandlung und die Homöopatika "Traumeel S", ein Antiphlogistikum, und "Atemaron N R30", ein Analgetikum/Antirheumatikum. Es ist nicht bekannt, ob diese Salben Hyperforin enthalten. Wegen der Instabilität von Hyperforin muß jedoch davon ausgegangen werden, daß Hyperforin nicht oder nur in geringen Mengen enthalten ist, wenn der Extrakt nicht unter Lichtschutz und Oxidationsschutz hergestellt, aufbewahrt und verarbeitet ist. In einem handelsüblichen Hypericum-Extrakt (Hyperforat) z.B. ist Hyperforin nicht nachweisbar.

Bei der topischen Anwendung und insbesondere bei der Behandlung entzündlicher Hauterkrankungen hat das bekannte Johanniskrautöl den Nachteil, daß es aufgrund seines Fettgehalts nur in begrenztem Umfang einsetzbar ist und sich beispielsweise für die Behandlung von Ekzemen nicht eignet. Darüber hinaus sind nur fettlösliche Bestandteile des Johanniskrauts in dem Öl enthalten, nicht jedoch beispielsweise Hypericin.

Zytostatika hemmen das Wachstum von insbesondere schnell wachsenden Zellen, wie sie in Tumoren und Leukämien vorliegen. Daher eignen sie sich als Chemotherapeutika gegen Krebs. Zahlreiche Chemotherapeutika sind bekannt, jedoch weisen diese Verbindungen auch Nachteile auf. Insbesondere treten bei der Behandlung mit bekannten Chemotherapeutika starke Nebenwirkungen auf, und die meisten Chemotherapeutika weisen nur gegenüber bestimmten Tumorzellinien eine befriedigende Aktivität auf.

Daher besteht weiterhin ein Bedürfnis nach zusätzlichen für die Krebsbehandlung geeigneten Verbindungen.

Eine Aufgabe der vorliegenden Erfindung besteht somit darin, ein weiteres Chemotherapeutikum zur Behandlung von Krebserkrankungen zur Verfügung zu stellen.

Eine weitere Aufgabe der vorliegenden Erfindung besteht darin, eine pharmazeutische Formulierung für das Chemotherapeutikum zur Verfügung zu stellen, die die genannten Nachteile nicht aufweist. Insbesondere soll sich die Formulierung an verschiedene Hautzustände anpassen lassen.

Es wurde nun überraschend gefunden, daß Hyperforin auf Tumorzellen eine proliferationshemmende Wirkung ausübt und Apoptose in Tumorzellen auslösen kann.

Die vorliegende Erfindung betrifft somit die Verwendung von Hyperforin zur Herstellung eines Arzneimittels zur Behandlung von Krebserkrankungen (Primärtumore und Metastasen) und/oder Präkanzerosen (Krebsvorstufen).

Unter Krebserkrankungen werden vorliegend insbesondere maligne Tumore sowie Lymphome und Leukämien verstanden.

Hyperforin ist jedoch auch gegen Metastasen, wie Metastasen des malignen Melanoms (schwarzer Hautkrebs) besonders wirksam.

Hyperforin hat sich auch gegenüber epithelialen Tumoren wie epithelialem Hautkrebs als wirksam erwiesen. Hierbei handelt es sich um einen langsam wachsenden Hautkrebs, der einer topischen Behandlung gut zugänglich ist. Der epitheliale Hautkrebs wird auch Spinaliom, Plattenepithelkarzinom oder Stachelzellkrebs genannt. Darüber hinaus eignet sich Hyperforin zur Behandlung von Krebsvorstufen (Präkanzerosen), wie beispielsweise solaren Präkanzerosen.

Darüber hinaus eignet sich Hyperforin zur Behandlung von Mamma-Karzinomen (Brustkrebs).

In der Praxis ist die Verwendung von Hyperforin insbesondere bei Lymphomen/Leukämien, schlecht operablen Tumoren und für die adjuvante Behandlung von Metastasen interessant. Insbesondere bei dem malignen Melanom zeigen alle bislang hierfür verfügbaren Therapien nur sehr mäßige Erfolge.

Für die Behandlung systemischer Tumore und von Metastasen kann Hyperforin intravenös verabreicht werden. Für die intravenöse Applikation kann der lyophilisierte oder getrocknete Wirkstoff beispielsweise frisch in physiologischer Kochsalzlösung aufgelöst und sofort injiziert bzw. infundiert werden. Der Wirkstoff kann jedoch auch oral, beispielsweise in Tablettenform verabreicht werden.

Hyperforin eignet sich aber auch für die lokale Anwendung beispielsweise durch intra- oder peritumorale Injektion oder Instillation (z.B. auch endoskopisch). Hierfür kann der Wirkstoff beispielsweise wie vorstehend für die intravenöse Applikation beschrieben bereitgestellt werden. Vorteilhaft kann der Wirkstoff jedoch auch durch epikutane Applikation beispielsweise in Cremeform angewandt werden, wobei sich diese Anwendungsform beispielsweise zur Behandlung von solaren Präkanzerosen besonders eignet.

Für die lokale, epikutane Applikation kann der Wirkstoff beispielsweise in Ethanol gelöst und in eine fette Salbengrundlage eingearbeitet werden. Diese kann okklusiv (unter Folie) beispielsweise für 24 Stunden auf den Tumor einwirken. Besonders bevorzugte Salben und Cremen werden im folgenden genauer beschrieben.

Bei der Aufbereitung des Wirkstoffs muß beachtet werden, daß es sich um eine lichtempfindliche Substanz handelt, die sich leicht zersetzt. Daher muß bei der Gewinnung, Lagerung und Verabreichung auf einen entsprechenden Lichtschutz geachtet werden.

Bei der erfindungsgemäßen Behandlung von Krebserkrankungen und/oder Präkanzerosen mit Hyperforin sollte die Hyperforinkonzentration am Wirkort so hoch sein, daß eine antiproliferative bzw. Apoptose-induzierende Wirkung eintritt. Die hierfür notwendige Konzentration kann je nach Art des behandelten Tumors variieren und vom Fachmann leicht bestimmt werden. Vorteilhaft ist beispielsweise bei intratumoraler Injektion eine Hyperforinkonzentration in der verabreichten Lösung von 50 µg/ml und bei epikutaner Applikation eine Wirkstoffkonzentration von 100 µg/ml. Bei systemischer Anwendung sollte der Wirkstoff in solchen Mengen injiziert werden, daß Plasmaspiegel von mindestens 50 µg/ml erreicht werden. Dies entspricht einer Hyperforinmenge von etwa 5 mg/kg Körpergewicht des Patienten.

Als ein weiterer Aspekt dieser Erfindung wurde gefunden, daß sich Hyperforin vorteilhaft in einer pharmazeutischen Formulierung in Form einer topischen Salbe oder Creme, die mindestens 15 µg/ml Hyperforin enthält, verabreichen läßt.

Die Salbe oder Creme sollte eine möglichst hohe Wirkstoffkonzentration enthalten, bevorzugt im Bereich von 0,02-20 mg/ml Hyperforin, bevorzugter im Bereich von 1-20 mg/ml und besonders bevorzugt etwa 10 mg/ml (1% Hyperforin).

Neben dem Wirkstoff Hyperforin kann die erfindungsgemäße Salbe oder Creme zusätzlich Hypericine als weitere Wirkstoffe enthalten. Die Konzentration der Hypericine in der Salbe oder Creme sollte dabei in Summe mindestens 15 µg/ml, bevorzugt 20-150 µg/ml betragen. Unter Hypericinen werden vorliegend Hypericin und dessen pharmazeutisch wirksamen Derivate verstanden. Hierzu gehört insbesondere Pseudohypericin, das sich von Hypericin dadurch unterscheidet, daß eine Methylgruppe durch Hydroxymethyl ersetzt ist.

Die genannten Wirkstoffe können entweder als Reinsubstanzen in die Salbe oder Creme eingebracht werden oder in Form eines Johanniskrautextrakts definierter Konzentration. Die erfindungsgemäße Salbe oder Creme umfaßt dabei bevorzugt außer Johanniskrautextrakt keine weiteren Pflanzenextrakte.

Beispielsweise eignet sich zur Herstellung der erfindungsgemäßen Salbe oder Creme ein Johanniskrautextrakt, der mindestens 200 µg/ml Hyperforin und mindestens 200 µg/ml Hypericine enthält. Bevorzugt enthält der verwendete Extrakt 200-100.000 µg/ml, insbesondere etwa 1000 µg/ml Hyperforin und 200-1000 µg/ml Hpericine. Bei diesen Wirkstoffkonzentrationen im Extrakt sollte die erfindungsgemäße Salbe oder Creme mindestens 5 Gew.-% des Extrakts enthalten. Vorteilhaft kann eine erfindungsgemäße Salbe beispielsweise etwa 15 Gew.-% des Extrakts enthalten und eine erfindungsgemäße Creme etwa 10 Gew.-% des Extrakts.

Der auf Hyperforin und Hypericin standardisierte Gesamtextrakt sollte ein ethanolischer oder ein mit Ethanol versetzter Extrakt sein. Hierbei kann es sich beispielsweise um im Handel erhältliche Gesamtextrakte (Tinkturen) handeln. Der Ethanolgehalt liegt bevorzugt zwischen 20 und 60 % v/v, bevorzugt bei 40-50% v/v. Diese Anforderungen erfüllt beispielsweise ein Gesamtextrakt der Firma Caelo, der erfindungsgemäß bevorzugt eingesetzt wird und der 240 µg/ml Hyperforin und 300 µg/ml Hypericin enthält.

Prinzipiell sind auch wäßrige Extrakte, $CO_2$-Extrakte oder Frischpflanzenauszüge geeignet, insofern sie die

Anforderungen an den Wirkstoffgehalt erfüllen.

Die qualitative und quantitative Analyse des für die erfindungsgemäße Salbe oder Creme verwendeten Johanniskrautextrakts erfolgt mittels Hochdruck-Flüssigkeits-Chromatographie (HPLC) (P. Maisenbacher et al., Planta Med. 58:351-354 (1992)). Zur Messung der Gesamthypericine wird das photometrische Verfahren nach dem "Deutschen-Arzneimittel-Codex" (DAC) verwendet.

Die erfindungsgemäße Salbe oder Creme kann neben dem Wirkstoff oder den Wirkstoffen verschiedene pharmazeutisch verträgliche Creme- bzw. Salbengrundlagen umfassen. Hierzu gehören beispielsweise weiße Vaseline, dickflüssiges Paraffin, Wollwachs, Ascorbylpalmitat, Glycerolmonostearat 60, Tocopherol (Vitamin E), Cetylalkohol, mittelkettige Triglyceride, gelbes Wachs, Propylenglycol, Macrogol-1000-glycerolmonostearat, Zitronensäure, Ascorbinsäure und andere Konservierungsstoffe und destilliertes Wasser.

Eine bevorzugte erfindungsgemäße Salbe umfaßt etwa 15 Gew.-% Johanniskrautextrakt sowie weiße Vaseline, dickflüssiges Paraffin, Wollwachs und Ascorbylpalmitat jeweils in geeigner Menge.

Eine bevorzugte erfindungsgemäße Creme umfaßt etwa 10 Gew.-% Johanniskrautextrakt sowie weiße Vaseline, Glycerolmonostearat 60, Cetylalkohol, mittelkettige Triglyceride, gelbes Wachs, Propylenglykol, Macrogol-1000-glycerolmonostearat, Zitronensäure und Wasser jeweils in geeigneter Menge.

Die vorliegende Erfindung betrifft auch ein Verfahren zur Herstellung einer topischen Salbe oder Creme, worin man Hyperforin und gegebenenfalls Hypericine oder einen Johanniskrautextrakt, der mindestens 200 μg/ml Hyperforin und mindestens 200 μg/ml Hypericine enthält, so mit üblichen pharmazeutisch verträglichen Hilfsstoffen vermischt, daß man eine Salbe oder Creme mit einem Mindestgehalt von 15 μg/ml Hyperforin und gegebenenfalls mindestens 15 μg/ml Hypericine erhält.

Bei Verwendung eines Johanniskrautextrakts zur Herstellung der erfindungsgemäßen Salbe oder Creme muß dieser zum Schutz des Hyperforins vor Oxidation bis zu seiner Verarbeitung unter Schutzgas (z.B. Argon) fest verschlossen und lichtgeschützt aufbewahrt werden.

Die erfindungsgemäße Salbe oder Creme eignet sich beispielsweise zur Behandlung von Krebserkrankungen, Präkanzerosen, entzündlichen Hauterkrankungen, Altershaut und bakteriellen Hautinfektionen. Die Salbe ist aufgrund der fetten Grundlage insbesondere bei trockenen, schuppenden Hautveränderungen, die mit Juckreiz oder Entzündungen einhergehen, indiziert. Der bevorzugte Wirkstoffgehalt der Salbe (15 Gew.-%) ist etwas höher als der bevorzugte Wirkstoffgehalt der Creme (10 Gew.-%), die sich aufgrund des amphiphilen Charakters besonders zur Behandlung akuter bis subakuter ekzematöser Hautveränderungen eignet.

Die erfindungsgemäße Salbe eignet sich somit insbesondere zur Behandlung von chronischen, auch superinfizierten Ekzemen, Exsikkationsekzemen, hyperkeratotischen Hand- und Fußekzemen, subakute bis chronische atopische Dermatitis (Neurodermitis), Lichen simplex, Kontaktekzemen, Prurigo simplex subacuta und andere Prurigo-Arten und Psoriasis vulgaris vom Plaque-Typ sowie der Altershaut.

Die Creme eignet sich insbesondere zur Behandlung akuter bis subakuter atopischer Dermatitis (Neurodermitits), akuten bis subakuten Kontaktekzemen, Psoriasis und der Altershaut sowie zur Nachbehandlung und Rezidivprophylaxe aller Ekzeme.

Die Salbe und die Creme können auch in der Veterinärmedizin beispielsweise zur Behandlung entzündlicher und infizierter Hautkrankheiten, wie Mastitis (Euterentzündung), verwendet werden.

Hyperforin wirkt ab Konzentrationen von 100 ng/ml proliferationshemmend auf humane Keratinozyten und Lymphozyten. Darüber hinaus konnte nachgewiesen werden, daß Hypericin proliferationshemmend auf Kerationzyten (HaCaT) und T-Zellen wirkt und in diesen Zellen Apoptose induzieren kann. Diese Wirkung wird teilweise durch die Bildung von Sauerstoffradikalen vermittelt.

Wegen der möglichen Photosensibilisierung aufgrund des optionalen Hypericin-Gehalts der erfindungsgemäßen Salbe oder Creme wurde untersucht, ob die lokale Anwendung des erfindungsgemäßen Hypericin-haltigen Präparats zu sonnenbrandähnlichen Erscheinungen führen kann. Diese Untersuchungen zeigten kein Sonnenbrandrisiko.

Die erfindungsgemäße Salbe oder Creme hat den Vorteil, daß sie von der Grundlage her an verschiedene Hautzustände angepaßt werden kann. So eignet sich die Creme besonders zur Behandlung akuter und subakuter Dermatosen, die Salbe zur Behandlung chronischer Dermatosen. Darüber hinaus können sowohl fettlösliche (Hyperforin) als auch wasserlösliche (Hypericin) Wirkstoffe des Johanniskrauts in eine Salben- bzw. Cremegrundlage eingearbeitet werden. Hierdurch kann eine überlegene Wirkung gegenüber dem bekannten Johanniskrautöl erreicht werden. Außerdem ist die Penetration der Wirkstoffe aus Creme- und Salbengrundlagen im Vergleich zu Ölen besser.

Durch die erfindungsgemäße Salbe oder Creme zur lokalen topischen Anwendung wird das Therapiespektrum entzündlicher Hautkrankheiten, wie z.B. Neurodermitis, entscheidend bereichert. Insbesondere besteht die Möglichkeit der Reduktion der Cortison-Therapie.

Die beiliegende Figur 1 zeigt die antiproliferative Wirkung von Hyperforin auf die Tumorzellinien HT144 (humane Melanom-Metastase), A431 (humanes Plattenepithelialkarzinom), Jurkat (humanes leukämisches Lymphom), 1F6 (humanes Melanom, Primärtumor) und MT450 (Ratten-Mammakarzinom) (Beispiel 1).

Figur 2 zeigt die Fähigkeit von Hyperforin Apoptose in den Tumorzellinien HT144 (humane Melanom-Metastase), A431 (humanes Plattenepithelialkarzinom), Jurkat (humanes leukämisches Lymphom), 1F6 (humanes Melanom, Primartumor) und MT450 (Ratten-Mammakarzinom) zu induzieren (Beispiel 2).

Figur 3 zeigt die Hyperforin-induzierte Apoptose durch selektive Aktivierung von Caspase 9 und 3 (Beispiel 5).

Figur 4 zeigt die Fähigkeit von Hyperforin in vivo das Tumorwachstum von Mamma-Karzinomzellen in ähnlicher Weise wie Taxol zu hemmen (Beispiel 6).

Figur 5 zeigt die proliferationshemmende Wirkung der erfindungsgemäßen Creme im Vergleich zu Johanniskrautöl nach Anwendung an gesunden Probanden in vivo (Beispiel 9).

Figur 6 zeigt die proliferationshemmende Wirkung von Hyperforin auf HaCaT-Zellen in vitro (Beispiel 13).

Figur 7 zeigt die proliferationshemmende Wirkung von Hyperforin auf PBMC in vitro (Beispiel 14).

Figur 8 zeigt die proliferationshemmende Wirkung einer auf Hyperforin standardisierten Creme und einer Hyperforin-Creme im Vergleich zum immunsuppressiven Effekt einer Sonnensimulator-Bestrahlung (zweifache MED). Als Kontrolle wurde unbehandelte Haut getestet (Beispiel 15).

Figur 9 zeigt die proliferationshemmende Wirkung von Hyperforin in vitro (Beispiel 16).

Die nachfolgenden Beispiele sollen die Erfindung näher erläutern. Für die Beispiele 1-6 wurde kommerziell erhältliches Hyperforin der Firma HWI Analytik, Rheinzabern, verwendet. Die Reinheit des Hyperforins betrug über 90%. In allen Versuchen wurde das Lösungsmittel DMSO in der maximalen verwendeten Konzentration getestet und zeigte keinerlei Effekte auf Proliferation oder Apoptoserate.

Beispiel 1

Die antiproliferative Wirkung von Hyperforin auf menschliche und Ratten-Tumorzellen wurde in vitro untersucht. Hierzu wurden Tumorzellen der Tumorzellinien **HT144** (humane Melanom-Metastase), A431 (humanes Plattenepithelialkarzinom), Jurkat (humanes leukämisches Lymphom), 1F6 (humanes Melanom, Primärtumor) und MT450 (Ratten-Mammakarzinom) in einer Konzentration von 1 x $10^5$ Zellen/ml in 96-Well-Mikrotiterplatten in 1640-RPMI mit 10% fetalem Kälberserum (FCS) mit 1% Penicillin/Streptomycin (alles Gibco) kultiviert (37°C, 5% $CO_2$). Es wurde frisch in DMSO gelöstes Hyperforin (HWI-Analytik) in verschiedenen Konzentrationen für 24h dazugegeben. Dann wurde 1 µCurie/well $^3$H-Thymidin (Amersham) zugegeben und die inkorporierte Radioaktivität nach 18h in einem Szintillationscounter (Canberra-Packard) gemessen. Die gemessene Radioaktivität ist proportional zur DNA-Replikation der Zellen.

Aus Figur 1 ist ersichtlich, daß die Hyperforin-Konzentration, bei der eine 50%-ige Wachstumshemmung der Zellen auftrat ($IC_{50}$), zwischen 5 und 15 µM lag.

Beispiel 2

Dieses Beispiel belegt, daß Hyperforin in Tumorzellen Apoptose, das heißt den sogenannten programmierten Zelltod, induziert. Die Auslösung von Apoptose in Tumorzellen ist für viele Zytostatika charakteristisch und spricht für die Wirksamkeit von Hyperforin als Zytostatikum.

HT144 (humane Melanom-Metastase), A431 (humanes Plattenepithelialkarzinom) Jurkat (humanes leukämisches Lymphom), 1F6 (humanes Melanom, Primärtumor) und MT450 (Ratten-Mammakarzinom) Tumorzellen wurden in einer Konzentration von 1 x $10^4$ Zellen/ml in 96-Well-Mikrotiterplatten kultiviert. Nach Vorinkubation der Zellen für 24 h wurde Hyperforin in den in Figur 2 angegebenen Endkonzentrationen zupipettiert. Die Zellen wurden anschließend lysiert und mit einem Cell Death Detection ELISA$^{PLUS}$ (Boehringer, Mannheim) auf niedermolekulare DNA-Fragmente untersucht. Hierzu wurde ein biotinylierter anti-Histon-Antikörper und ein Peroxidase-gekoppelter anti-DNA-Antikörper verwendet und der Anteil an niedermolekularer DNA durch Bestimmung der Peroxidaseabsorption bei 405 nm bestimmt.

Die Ergebnisse sind in Figur 2 wiedergegeben, die die Extinktion bei 405 nm nach Abzug der unbehandelten Kontrolle darstellt. Es zeigt sich, daß Hyperforin dosisabhängig in allen Tumorzellinien Apoptose induziert.

Beispiel 3

Mittels eines Zytotoxizitätsassays wurde der toxische Effekt von Hyperforin bei verschiedenen Konzentrationen auf die in den Beispielen 1 und 2 verwendeten Tumorzellinien HT144, A431 und Jurkat untersucht. Hierzu wurde die Membranintegrität durch Trypanblau-Exklusion bestimmt. Das Ergebnis ist in Tabelle 1 wiedergegeben, wobei die Angaben in % der unbehandelten Zellen erfolgen. Toxische Effekte waren kaum nachweisbar, dies bestätigt die spezifische Induktion von Apoptose durch Hyperforin.

Tabelle 1

| Hyperforin (µg/ml) | Trypanblau-Exklusion (% der Zellen) | | |
|---|---|---|---|
| | HT144 | A431 | Jurkat |
| 0 | 100 | 100 | 100 |
| 2,5 | 100 | 100 | 100 |
| 5 | 100 | 100 | 100 |
| 10 | 100 | 100 | 100 |
| 20 | 100 | 95 | 100 |
| 40 | 90 | 90 | 100 |
| 80 | 80 | 90 | 100 |

Beispiel 4

Das folgende Beispiel belegt die apoptotische DNA-Fragmentierung durch Hyperforin und das bekannte Zytostatikum Taxol als Vergleichssubstanz. Die DNA-Fragmentierung wurde mittels DNA-Gelelektrophorese bestimmt. Als Tumorzellinie wurde Jurkat (Leukämie) eingesetzt.

Jeweils 1 x $10^6$ Zellen wurden unbehandelt oder mit Hyperforin (40 µM) bzw. Taxol (10 µM) bei 37°C inkubiert. Apoptotische DNA-Fragmente wurden mittels NP 40-Lyse isoliert. Die Zellen wurden nach 4 Stunden bzw. nach 24 Stunden gewaschen und pelletiert. Das Zellpellet wurde für 10 Sek. mit Lysepuffer inkubiert (1% NP 40, Sigma; 20 mM EDTA, Sigma; 50 mM Tris-HCl, Sigma). Die Lysate wurden mit 1% SDS (Sigma) gemischt, 2 h mit Rnase (5 µg/µl) (Boehringer) bei 56°C inkubiert und mit Proteinase K (Sigma) (2,5 µg/µl) für 2 h bei 37°C verdaut. Nach Zugabe von 10 M Ammoniumacetat wurde die DNA mit 100% Ethanol bei -20°C gefällt und mittels Gelelektrophorese auf 1% Agarosegelen analysiert.

Die Ergebnisse sind in Tabelle 2 wiedergegeben. Es zeigte sich, daß Hyperforin im Vergleich zu Taxol schneller Apoptose in Turmorzellen induziert, da diese bereits nach 4 Stunden voll ausgebildet war.

Tabelle 2

| | Unbehandelte Zellen | Hyperforin (40 µM) | Taxol (10 µM) |
|---|---|---|---|
| 4 Stunden | - | ++ | + |
| 24 Stunden | - | ++ | ++ |

++ = stark positiv
+ = positiv
- = negativ

Beispiel 5

Um einen möglichen Wirkmechanismus der Apoptose-Induktion durch Hyperforin nachzuweisen, wurde die Aktivität unterschiedlicher Caspasen in Tumorzellinien untersucht. Die Aktivierung von Caspasen kann durch verschiedene Signaltransduktions-Mechanismen erfolgen und führt über die Aktivierung von Effektor-Caspasen (z.B. Caspase 3) zur Induktion des programmierten Zelltodes. Die Aktivität der Upstream-Caspase 9, der Downstream-Caspase 8 und der Effektor-Caspase 3 wurden mittels kommerziell erhältlicher Caspase-Kits (R&D Systems) gemessen. MT450 Zellen wurden in einer Konzentration von 1 Mio. Zellen/ml ohne oder mit Hyperforin (Endkonz. 50 µM) für 24h inkubiert. Danach wurden die Zellen abzentrifugiert, der Überstand abgenommen und die Zellen mittels Lysepuffer lysiert. Das Zellysat wurde jeweils mit einem für die Caspase spezifischen Substrat inkubiert und das an einem Farbstoff gekoppelte Spaltprodukt photometrisch im ELISA-Reader detektiert.

Die Beeinflussung der Caspase-Aktivität ist in Figur 3 relativ zur unbehandelten Kontrolle (Kontrolle = 1) dargestellt. Es zeigt sich, daß Hyperforin zu einer Aufregulation von Caspase 9 und Caspase 3 führt, nicht jedoch zur Aktivierung von Caspase 8.

Beispiel 6

In vivo wurde der Effekt von Hyperforin auf das Wachstum von MT450-Zellen (Mamma-Karzinom) bei Ratten untersucht. Zum Vergleich wurde eine Behandlung mit gleichen Konzentrationen des Zytostatikums Taxol durchgeführt. Jede Behandlungsgruppe bestand aus 8 Versuchstieren, die Behandlung erfolgte verdeckt (Blindversuch). Pro Tier

wurden je 1 Mio. Tumorzellen injiziert. Nach 3 Wochen wurde mit der intratumoralen Injektion begonnen. Die Injektion erfolgte täglich mit je 100 µl des Lösungsmittels (10% DMSO in PBS), des Hyperforins (500 µg in 10% DMSO/PBS) oder des Taxols (500 µg in 10% DMSO/PBS). Das Tumorvolumen wurde planimetrisch vom Beginn der Tumorinjektion (Tag 0) bis zum Abschluß der Behandlung (Tag 30) 8 mal gemessen und in Figur 4 als Wachstumskurve (Mittelwert +/- Standardabweichung) aufgetragen. Es zeigt sich, daß Hyperforin im selben Maße wie Taxol in vivo signifikant das Wachstum von MT405-Zellen hemmt.

Beispiel 7

Eine Johanniskrautsalbe wurde nach folgender Rezeptur hergestellt (Angaben in Gew.-%):

| | |
|---|---|
| Weiße Vaseline | 50.0 |
| Dickflüssiges Paraffin | 9.0 |
| Wollwachs | 25.0 |
| Ascorbylpalmitat | 1.0 |
| Johanniskrautextrakt | 15.0 |
| | 100 |

Die Vaseline, das dickflüssige Paraffin und das Wollwachs wurden im Wasserbad auf 60°C erwärmt und gemischt. Man ließ unter Rühren abkühlen, wobei das Ascorbylpalmitat eingearbeitet wurde, und nach dem Abkühlen wurde das Johanniskrautextrakt (Gesamtextrakt der Firma Caelo (Hyperforin 240 µg/ml, Hypericin 300 µg/ml) eingearbeitet. Die so erhaltene Salbe enthielt 36 µg/ml Hyperforin und 45 µg/ml Hypericin.

Beispiel 8

Nach folgender Rezeptur wurde eine Johanniskrautcreme hergestellt (Angaben in Gew.-%):

| | |
|---|---|
| Weiße Vaseline | 20.0 |
| Glycerolmonostearat 60 | 4.0 |
| Cetylalkohol | 6.0 |
| Mittelkettige Triglyceride | 8.0 |
| Gelbes Wachs | 4.0 |
| Propylenglykol | 10.0 |
| Macrogol-1000-glycerolmonostearat | 7.0 |
| Zitronensäure | 1.0 |
| Destilliertes Wasser | 30.0 |
| Johanniskrautextrakt | 10.0 |
| | 100 |

Die Vaseline, das Glycerolmonostearat 60, der Cetylalkohol, die mittelkettigen Triglyceride und das gelbe Wachs wurden im Wasserbad auf 60°C erhitzt und gemischt. Separat wurde das Macrogol-1000-glycerolmonostearat, das Propylenglykol, das Wasser und die Zitronensäure im Wasserbad auf 60°C erhitzt und dann in die erste Mischung eingearbeitet. Es wurde bis zum Erkalten gerührt und anschließend wurde der Johanniskrautextrakt (Gesamtextrakt der Firma Caelo (Hyperforin 240 µg/ml, Hypericin 300 µg/ml)) eingearbeitet. Die so erhaltene Creme enthielt 24 µg/ml Hyperforin und 30 µg/ml Hypericin.

Beispiel 9

Zur Herstellung einer Hyperforin-Salbe bzw. -Creme mit höherer Wirkstoffkonzentration wurde ein mit Neutralöl versetzter Extrakt der Firma Flavix (Rehlingen) mit einem Hyperforingehalt von 20 Gew.-% (20 g/100 g) verwendet. Hierzu wurden 5 g des Extrakts in 10 ml 70%-igem Ethanol aufgelöst, um eine Ausgangslösung mit einer Hyperforinkonzentration von 100 mg/ml zu erhalten. Diese Ausgangslösung wurde an Stelle des Johanniskrautextrakts der Firma Caelo in der in Beispiel 7 bzw. 8 beschriebenen Weise in eine Salben- bzw. Cremegrundlage eingearbeitet. Es wurden Salben und Cremen folgender Rezepturen hergestellt (Angaben in Gew.-%):

a) Salbe

| | 1% Hyperforin | 0,1% Hyperforin |
|---|---|---|
| Weiße Vaseline | 50,0 | 50,0 |
| Dickflüssiges Paraffin | 9,0 | 9,0 |
| Wollwachs | 30,0 | 39,0 |
| Ascorbylpalmitat | 1,0 | 1,0 |
| Ausgangslösung | 10,0 | 1,0 |
| | 100,0 | 100,0 |

b) Creme

| | 1% Hyperforin | 0,1% Hyperforin |
|---|---|---|
| Weiße Vaseline | 20,0 | 20,0 |
| Glycerolmonostearat | 4,0 | 4,0 |
| Cetylalkohol | 6,0 | 6,0 |
| Mittelkettige Triglyceride | 8,0 | 17,0 |
| Gelbes Wachs | 4,0 | 4,0 |
| Propylenglykol | 10,0 | 10,0 |
| Macrogol-1000-glycerolmonostearat | 7,0 | 7,0 |
| Zitronensäure | 1,0 | 1,0 |
| Destilliertes Wasser | 30,0 | 30,0 |
| Ausgangslösung | 10,0 | 1,0 |
| | 100,0 | 100,0 |

Beispiel 10

Die immunomodulatorischen Wirkungen der erfindungsgemäßen Präparation auf die Haut wurden ex vivo am Menschen (3x4 Probanden) nach Anwendung der Johanniskrautsalbe aus Beispiel 7 im Vergleich zu Johanniskrautöl untersucht. Hierzu wurden bei freiwilligen Probanden unterschiedlich behandelte Hautproben entnommen und es wurde untersucht, ob die Fähigkeit epidermaler Langerhans-Zellen, Antigen zu präsentieren, beeinflußt wird.

Im einzelnen wurde folgende MECLR (mixed epidermal cell leukocyte reaction) durchgeführt: Bei jeweils 4 Probanden wurden an den Unterarm-Beugeseiten runde Testfelder mit 2 cm Durchmesser mit Johanniskrautöl, Johanniskrautcreme oder der Behandlungsgrundlage behandelt und der Effekt im Vergleich zu unbehandelter Haut untersucht. 100 µl der Testsubstanzen wurden für 24h in Epikutantestkammern appliziert. Danach wurden die Rückstände entfernt und mittels eines Vakuums epidermale Saugblasen erzeugt. Das Blasendach wurde steril mit dem Skalpell abpräpariert und es wurde durch Trypsinbehandlung eine Epidermalzellsuspension (EC) hergestellt. 50.000 EC wurden mit 150.000 T-Zellen (TC) in RPMI-1640 mit 10% fötalem Kälberserum (FCS) mit 1% Penicillin/Streptomycin (alles Gibco) in 96-well Flachboden Mikrotiterplatten (Greiner) für 6 Tage kokultiviert (37°C, 5% $CO_2$). Dann wurde 1 µCurie/well $^3$H-Thymidin (Amersham) zugegeben und die inkorporierte Radioaktivität in einem Szintillationscounter (Canberra-Packard) gemessen. Die gemessene Radioaktivität ist proportional zur DNA-Replikation der Zellen.

Diese Untersuchungen zeigten, daß es durch die erfindungsgemäße Johanniskrautsalbe zu einer Proliferationshemmung kommt. Bei der Anwendung von Johanniskrautöl hingegen kommt es zu einer Proliferationssteigerung (Figur 5).

Diese Ergebnisse sprechen für eine antientzündliche Wirkung der erfindungsgemäßen Johanniskrautsalbe,

die beim Öl nicht nachweisbar war.

Beispiel 11

Bei einem Patienten mit Ekzem wurde in einem Halbseitenversuch ein Unterschenkel für zwei Wochen mit Johanniskrautöl, der andere mit der erfindungsgemäßen Johanniskrautsalbe gemäß Beispiel 7 behandelt. Der mit der Salbe behandelte Unterschenkel war bei der Kontrolle sehr gut gebessert, der mit dem Öl behandelte Unterschenkel eher verschlechtert.

Beispiel 12

Es wurden 4 Patienten mit verschiedenen lokalisierten Ekzemformen behandelt. Nach Aufklärung und Einwilligung erfolgte eine Fotodokumention und die Patienten führten über 2 Wochen eine Monotherapie mit der erfindungsgemäßen Salbe durch. Danach wurde eine erneute Fotokokumentation durchgeführt. Bei zwei Patienten kam es zu einer vollständigen Abheilung, bei den anderen Patienten zu einer deutlichen Besserung des Befundes. Die Ergebnisse dieser Behandlung sind in Tabelle 3 zusammengefaßt.

Tabelle 3

| Nr. | Geschlecht | Diagnose | Behandlung | Therapieerfolg |
|---|---|---|---|---|
| 1 | weiblich | Beugenekzem | Hypericum-Creme | abgeheilt |
| 2 | männlich | Handekzem | Hypericum-Creme | gebessert |
| 3 | weiblich | Neurodermitis Arme | Hypericum-Creme | abgeheilt |
| 4 | weiblich | Prurigo Unterschenkel | Hypericum-Creme | gebessert |

Beispiel 13

Dieses Beispiel zeigt die proliferationshemmende Wirkung von Hyperforin auf Keratinozyten. HaCaT-Zellen wurden in Keratinozyten-Medium mit 10% fötalem Kälberserum (FCS) mit 1 % Penicillin/Streptomycin (alles Gibco) kultiviert (37° C, 5% $CO_2$). Subkonfluente Kulturen wurden mit EDTA-Trypsin (Gibco) abgelöst, 3x in PBS gewaschen und in einer Dichte von 20.000/well in 96-well Flachboden Mikrotiterplatten (Greiner) weiter für 24h kultiviert (bis zur Adhärenz). Danach wurde frisch in DMSO gelöstes Hyperforin (HWI-Analytik) für 24h dazugegeben. Dann wurde 1 µCurie/well $^3$H-Thymidin (Amersham) zugegeben und die inkorporierte Radioaktivität in einem Szintillationscounter (Canberra-Packard) gemessen. Die gemessene Radioaktivität ist proportional zur DNA-Replikation der Zellen. Die Ergebnisse für Hyperforinkonzentrationen von 0 bis 100 µg/ml sind in Figur 6 wiedergegeben, wobei cpm counts per minute bedeutet. Es zeigt sich, daß es bei einer Hyperforinkonzentration von 100 µg/ml zu einer praktisch vollständigen Proliferationshemmung kommt.

Beispiel 14

Dieses Beispiel zeigt die proliferationshemmende Wirkung von Hyperforin auf mononukleäre Zellen des peripheren Blutes (PBMC). PBMC wurden durch Dichtegradient-Zentrifugation mit Ficoll (Seromed) aus heparinisiertem Blut gewonnen. Die PBMC wurden 3x in PBS gewaschen und in RPMI-1640 mit 10% fötalem Kälberserum (FCS) mit 1% Penicillin/Streptomycin (alles Gibco) in einer Dichte von 200.000/well in 96-well Flachboden Mikrotiterplatten (Greiner) für 24h kultiviert (37° C, 5% $CO_2$). Danach wurden die Zellen mit 1 µg/ml Phytohämagglutinin (PHA) (Wellcome) stimuliert und es wurde frisch in DMSO gelöstes Hyperforin (HWI-Analytik) für 24h dazugegeben. Dann wurde 1 µCurie/well $^3$H-Thymidin (Amersham) zugegeben und die inkorporierte Radioaktivität in einem Szintillationscounter (Canberra-Packard) gemessen. Die gemessene Radioaktivität ist proportional zur DNA-Replikation der Zellen. Die Ergebnisse für Hyperforinkonzentrationen von 0 bis 100 µg/ml sind in Figur 7 wiedergegeben, wobei cpm counts per minute bedeutet. Es zeigt sich, daß bereits geringe Konzentrationen von Hyperforin proliferationshemmend auf PBMC wirken und es bei einer Konzentration von 100 µg/ml zu einer praktisch vollständigen Proliferationshemmung kommt.

Beispiel 15

Dieses Beispiel zeigt eine immunmodulatorische Wirkung von Hyperforin-Creme mit hohem Hyperforinanteil in vivo. Es konnte durch Experimente mit aufgereinigtem Hyperforin gezeigt werden, daß diese immunmodulatorische

Wirkung auf Hyperforin zurückzuführen ist.

Die immunmodulatorische Wirkung der erfindungsgemäßen Creme wurde ex vivo am Menschen (je 4 Probanden) getestet. Hierzu wurden unterschiedlich behandelte Hautproben entnommen und es wurde untersucht, ob die Fähigkeit epidermaler Langerhans-Zellen, Antigen zu präsentieren beeinflußt wird. Im einzelnen wurde dies in einer MECLR (mixed epidermal cell leukocyte reaction) untersucht: Bei je 4 Probanden wurden an der Unterarm-Beugeseite runde Testfelder, mit 2 cm Durchmesser mit Hypericum-Creme (mit 24 μg/ml Hyperforin und 30 μg/ml Hypericin), mit Hyperforin-Creme (24 μg/ml) oder mit Sonnensimulator-Bestrahlung (144 J/$cm^2$) behandelt. Als Kontrollen dienten unbehandelte Haut und Anwendung des Vehikels ohne Wirkstoffe. 100 μl der Testsubstanzen wurden für 24h in Epikutantestkammern appliziert. Danach wurden die Rückstände entfernt und mittels eines Vakuums eine epidermale Saugblase erzeugt. Das Blasendach wurde steril mit dem Skalpell abpräpariert und es wurde durch Trypsinbehandlung eine Epidermalzellsuspension (EC) hergestellt. 50.000 EC wurden mit 150.000 TC (T-Zellen) in 1640-RPMI mit 10% fötalem Kälberserum (FCS) mit 1% Penicillin-Streptomycin in 96-well Flachboden Mikrotiterplatten (Greiner) für 6 Tage kokultiviert (37°C, 5% $CO_2$). Dann wurde 1 μCurie/well $^3$H-Thymidin (Amersham) zugegeben und nach 18h die inkorporierte Radioaktivität in einem Szintillationscounter (Canberra-Packard) gemessen. Die gemessene Radioaktivität ist proportional zur DNA-Replikation der Zellen.

Die Ergebnisse (Figur 8) zeigen, daß die Hyperforin-haltige Johanniskrautcreme und die Hyperforin-Creme eine signifikante Hemmung der Proliferation in der Größenordnung der Bestrahlung mit dem Sonnensimulator bewirken.

Beispiel 16

Dieses Beispiel zeigt die proliferationshemmende Wirkung von Hyperforin in vitro. In allen Fällen wurde reines Hyperforin der Firma HWI-Analytik (Rheinzabern) verwendet. Die Reinheit des Hyperforins betrug > 90%. In allen in vitro-Versuchen wurde das Lösungsmittel DMSO in der maximalen verwendeten Konzentration getestet und zeigte keinen Effekt auf Proliferation und Vitalität der Zellen. Es wurden normale Hautproben gesunder Probanden entnommen, diese wurden in vitro mit Hyperforin inkubiert und es wurde untersucht, ob die Fähigkeit epidermaler Langerhans-Zellen, Antigen zu präsentieren beeinflußt wird.

Dies wurde wie in Beispiel 14 in einer MECLR (mixed epidermal cell leukocyte reaction) untersucht: Bei je 4 Probanden wurden an der Unterarm-Beugeseite mittels eines Vakuums epidermale Saugblasen erzeugt. Das Blasendach wurde steril mit dem Skalpell abpräpariert und es wurde durch Trypsinbehandlung eine Epidermalzellsuspension (EC) hergestellt. Es wurde jeweils ein Teil der EC oder TC für 24h mit 24 μg/ml Hyperforin inkubiert. Danach wurden die Zellen gewaschen, und es wurden 50.000 EC mit 150.000 TC (T-Zellen) in 1640-RPMI mit 10% fötalem Kälberserum (FCS) mit 1% Penicillin-Streptomycin in 96-well Flachboden Mikrotiterplatten (Greiner) für 6 Tage kokultiviert (37°C, 5% $CO_2$). Dann wurde 1 μCurie/well $^3$H-Thymidin (Amersham) zugegeben und nach 18h die inkorporierte Radioaktivität in einem Szintillationscounter (Canberra-Packard) gemessen. Die gemessene Radioaktivität ist proportional zur DNA-Replikation der Zellen.

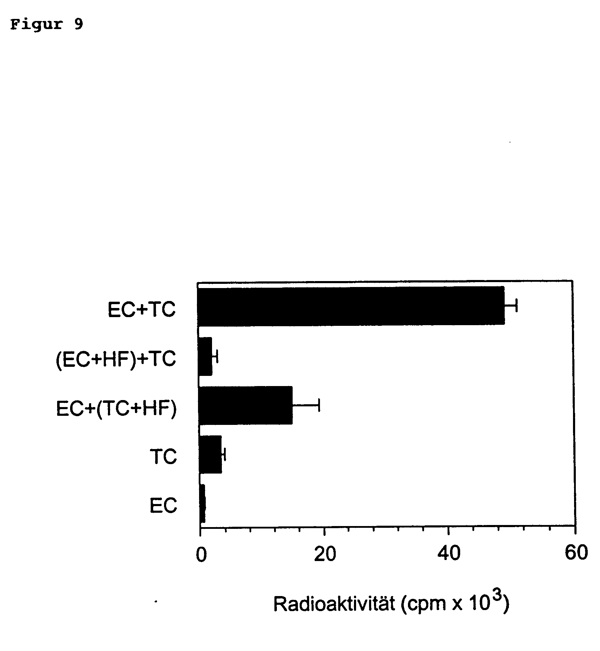

Die Ergebnisse (Figur 9) zeigen, daß Hyperforin sowohl auf EC als auch auf TC eine signifikante Hemmung der Proliferation bewirkt.

**Patentansprüche**

1. Verwendung von Hyperforin zur Herstellung eines Arzneimittels zur Behandlung von Krebserkrankungen und/oder Präkanzerosen.

2. Verwendung nach Anspruch 1 zur Behandlung von Lymphomen und/oder Leukämien.

3. Verwendung nach Anspruch 1 zur Behandlung von Metastasen, insbesondere Melanom-Metastasen.

4. Verwendung nach Anspruch 1 zur Behandlung von epithelialen Tumoren und/oder epithelialen Präkanzerosen.

5. Topische Salbe oder Creme, enthaltend mindestens 15 μg/ml Hyperforin.

6. Salbe oder Creme nach Anspruch 5, enthaltend 0,02-20 mg/ml, bevorzugt 1-20 mg/ml Hyperforin.

7. Salbe oder Creme nach einem der Ansprüche 5 oder 6, enthaltend zusätzlich mindestens 15 μg/ml Hypericine.

8. Salbe oder Creme nach Anspruch 7, enthaltend mindestens 5 Gew.-% Johanniskrautextrakt, der mindestens 200

µg/ml Hyperforin und mindestens 200 µg/ml Hypericine enthält.

9. Salbe oder Creme nach Anspruch 8, worin der Johanniskrautextrakt 200-100.000 µg/ml, bevorzugt etwa 1000 µg/ml Hyperforin und 200-2000 µg/ml Hypericine enthält.

10. Salbe oder Creme nach Anspruch 8 oder 9, worin der Johanniskrautextrakt 20-60 % v/v Ethanol enthält.

11. Salbe nach einem der Ansprüche 7-10, enthaltend etwa 15 Gew.-% Johanniskrautextrakt sowie eine übliche Salbengrundlage.

12. Creme nach einem der Ansprüche 7-10, enthaltend etwa 10 Gew.-% Johanniskrautextrakt sowie eine übliche Cremegrundlage.

13. Verfahren zur Herstellung einer topischen Salbe oder Creme nach einem der Ansprüche 5-12, worin man Hyperforin und gegebenenfalls Hypericine oder einen Johanniskrautextrakt, der mindestens 200 µg/ml Hyperforin und mindestens 200 µg/ml Hypericine enthält, so mit üblichen pharmazeutisch verträglichen Hilfsstoffen vermischt, daß man eine Salbe oder Creme mit einem Mindestgehalt von 15 µg/ml, bevorzugt 1-20 mg/ml Hyperforin und gegebenenfalls 15 µg/ml Hypericine erhält.

14. Verwendung einer Salbe oder Creme nach einem der Ansprüche 5-12 zur Behandlung von Krebserkrankungen und/oder Präkanzerosen.

15. Verfahren zur Behandlung von Krebserkrankungen und/oder Präkanzerosen durch Verabreichung von Hyperforin.

16. Verfahren zur Behandlung von Krebserkrankungen und/oder Präkanzerosen durch Auftragen einer Salbe oder Creme nach einem der Ansprüche 5-12 auf die Haut.

Figur 1

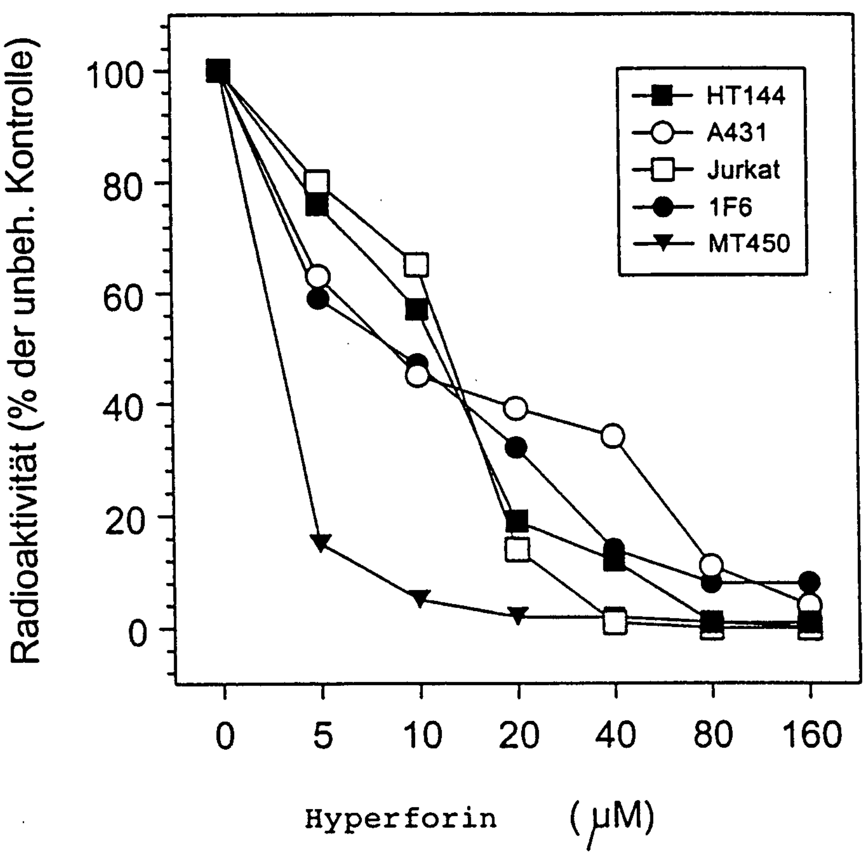
Radioaktivität (% der unbeh. Kontrolle)
100
80
60
40
20
0
0
5
10
20
40
80
160
HT144
A431
Jurkat
1F6
MT450
Hyperforin (μM)

Figur 2

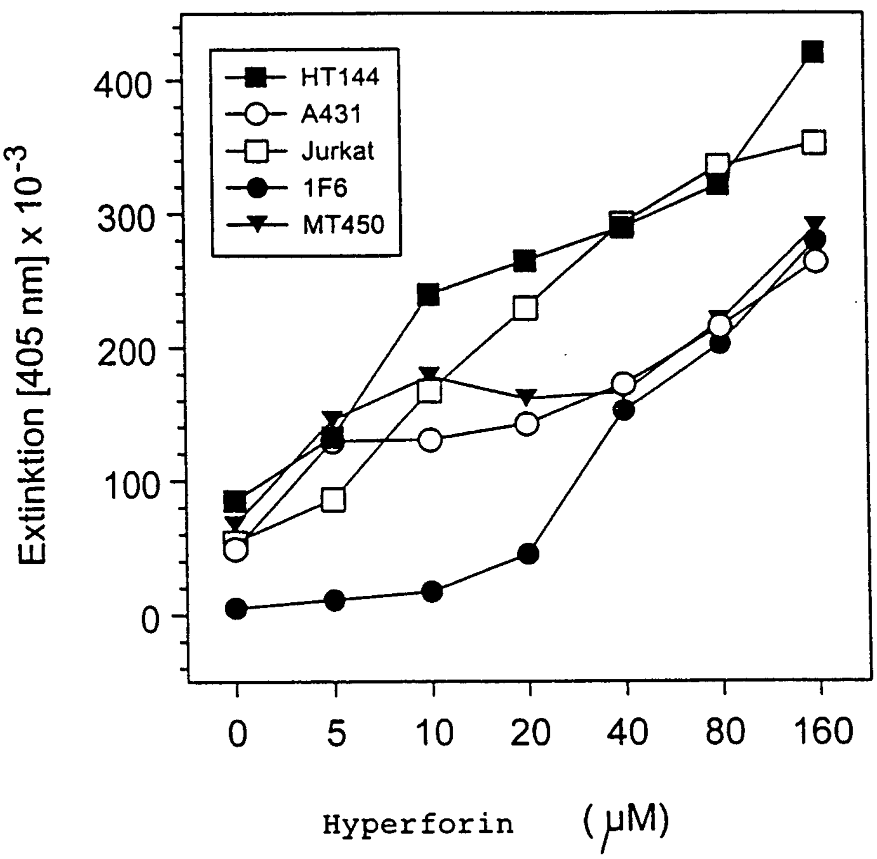

400
300
200
100
0
Extinktion [405 nm] x 10-3
HT144
A431
Jurkat
1F6
MT450
0
5
10
20
40
80
160
Hyperforin (µM)

Figur 3

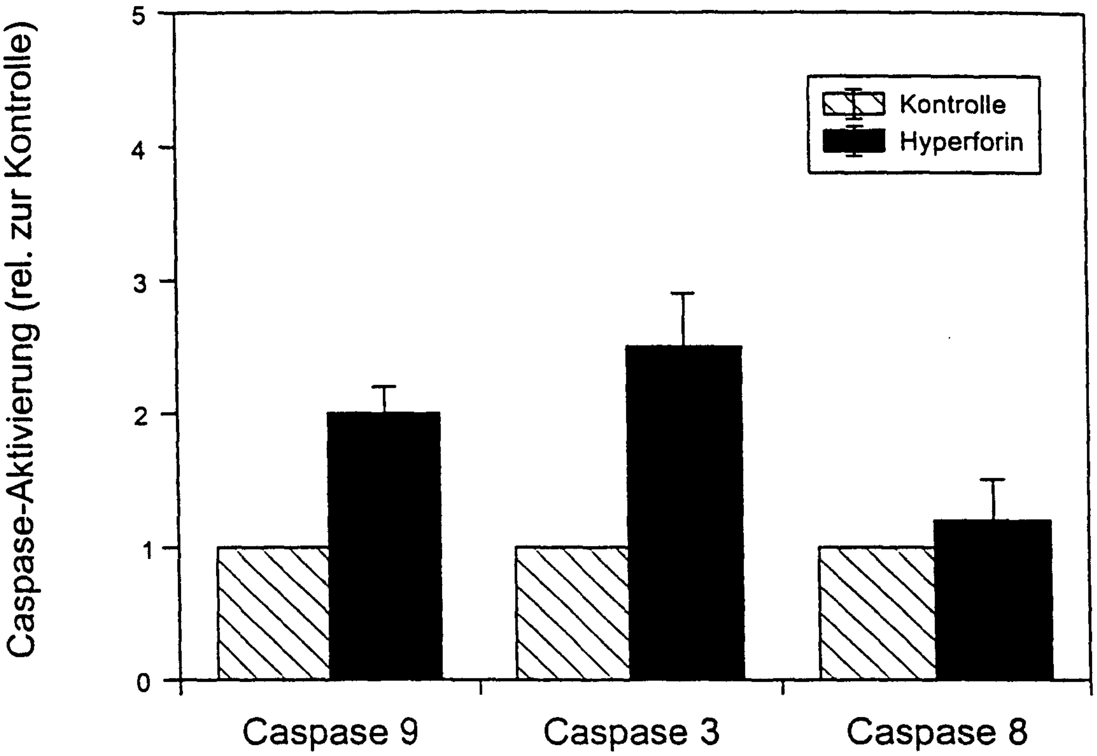
Caspase-Aktivierung (rel. zur Kontrolle)
0
1
2
3
4
5
Kontrolle
Hyperforin
Caspase 9
Caspase 3
Caspase 8

Firgur 4

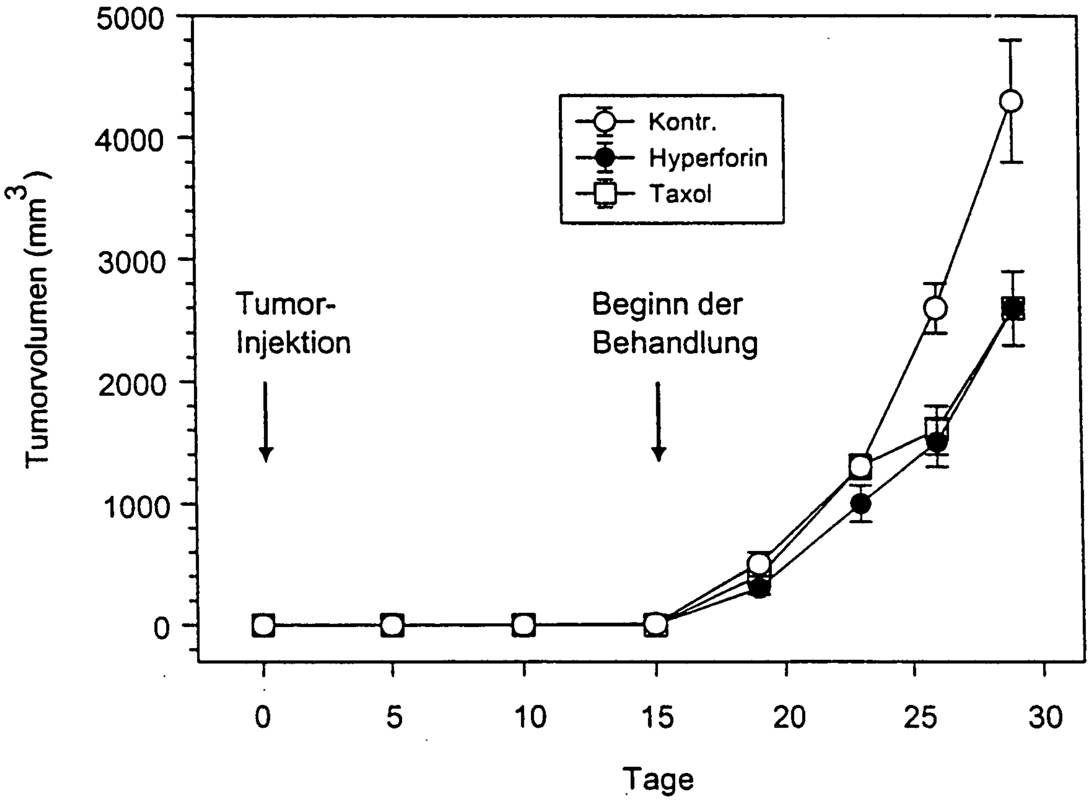
5000
4000
3000
2000
1000
0
Tumorvolumen (mm3)
Kontr.
Hyperforin
Taxol
Tumor-
Injektion
Beginn der
Behandlung
0
5
10
15
20
25
30
Tage

Figur 5

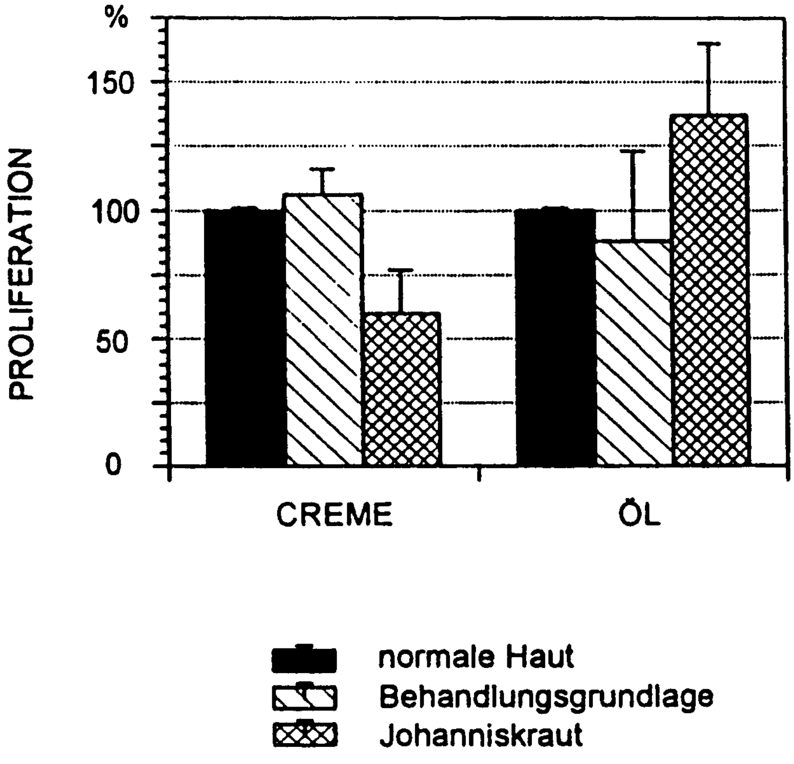
%
150
100
50
0
PROLIFERATION
CREME
ÖL
normale Haut
Behandlungsgrundlage
Johanniskraut

Figur 6

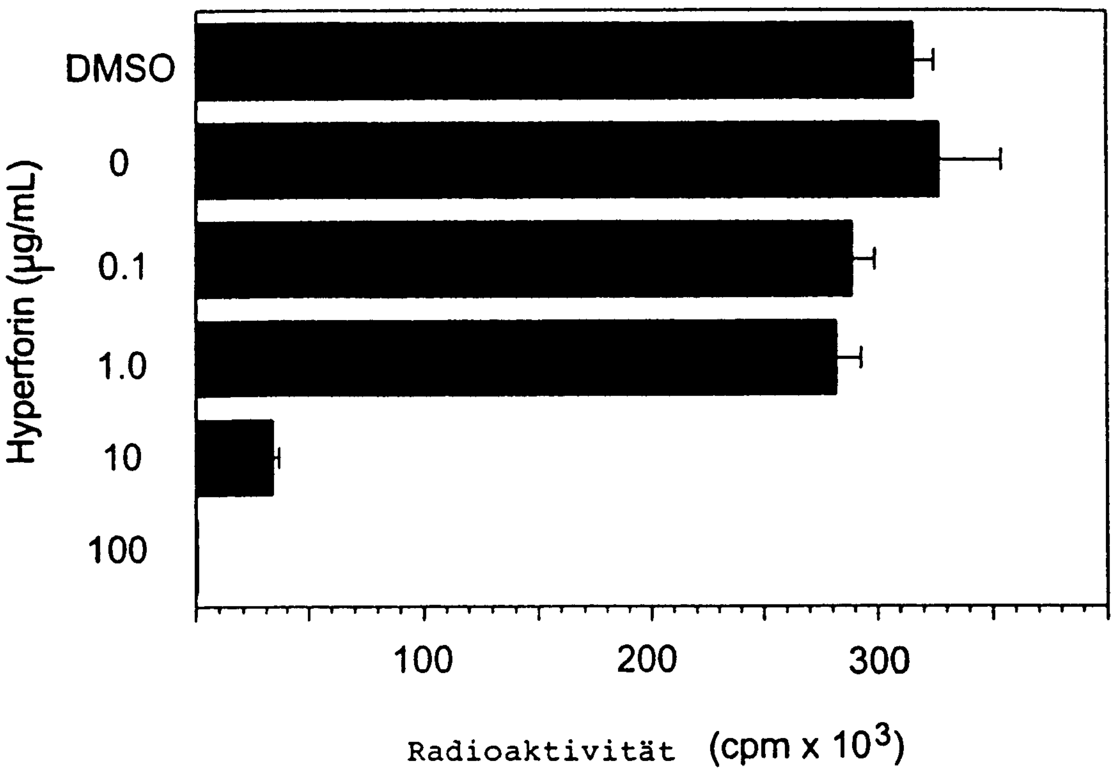
DMSO
0
0.1
1.0
10
100
Hyperforin (µg/mL)
100
200
300
Radioaktivität (cpm x $10^3$)

Figur 7

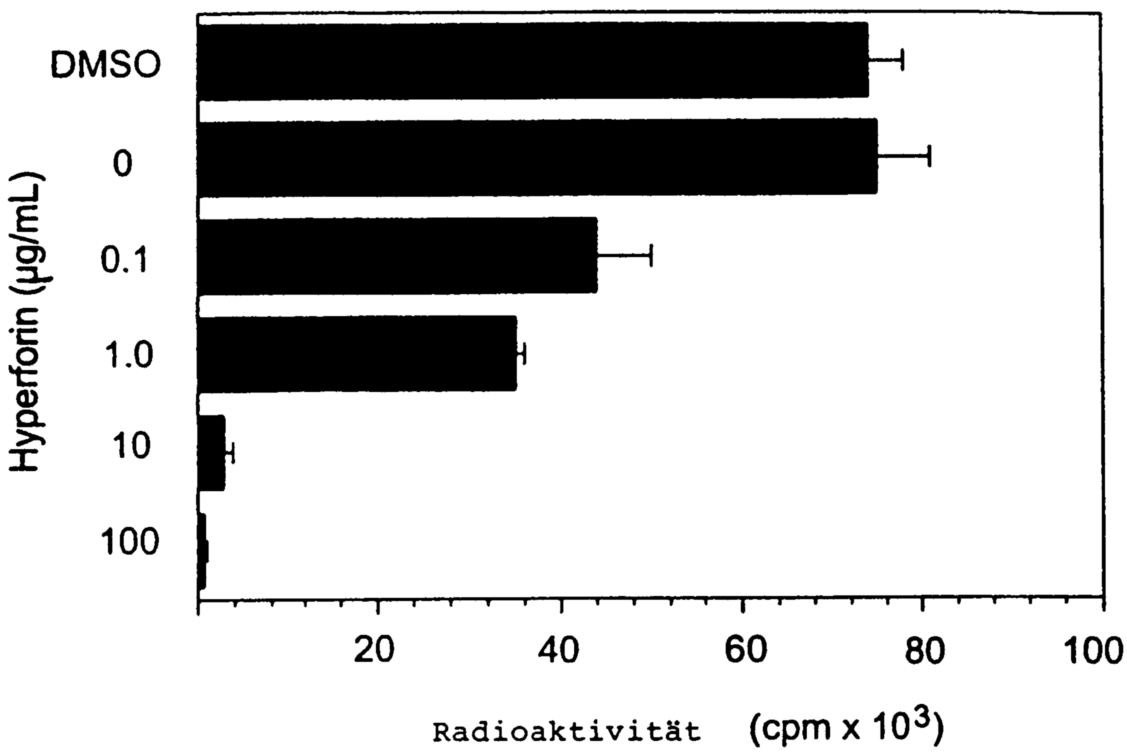
DMSO
0
0.1
1.0
10
100
Hyperforin (µg/mL)
20
40
60
80
100
Radioaktivität (cpm x 10³)

Figur 8

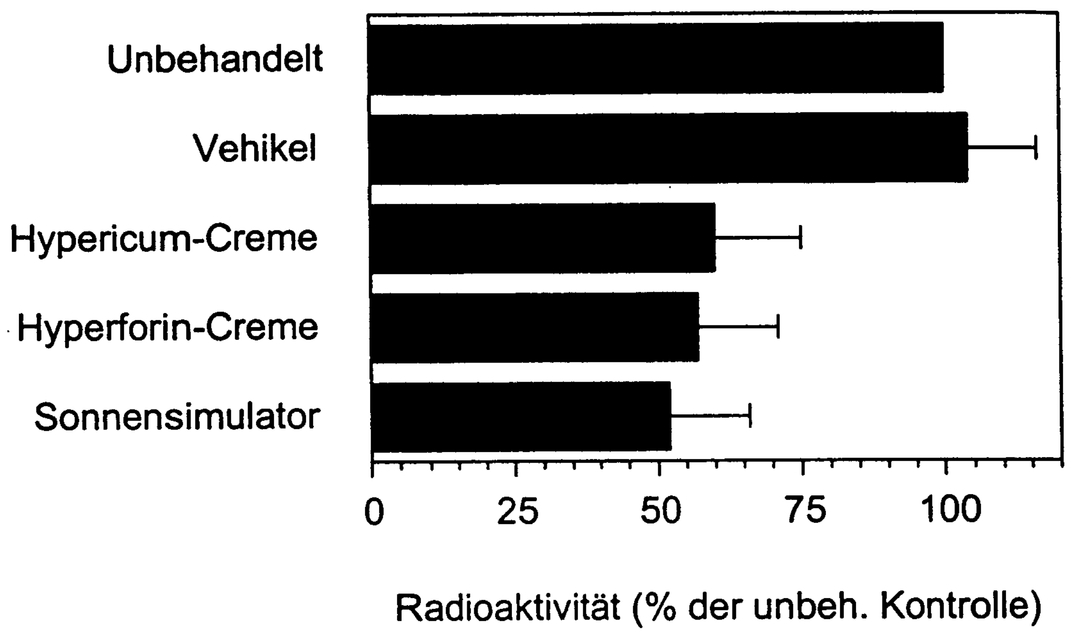
Unbehandelt
Vehikel
Hypericum-Creme
Hyperforin-Creme
Sonnensimulator
0
25
50
75
100
Radioaktivität (% der unbeh. Kontrolle)

Figur 9